# EUROPEAN PATENT SPECIFICATION

(11) **EP 4 577 276 B1**
(45) Date of publication and mention of the grant of the patent: **18.02.2026**
(21) Application number: 23825230.8
(22) Date of filing: 06.12.2023
(51) Int. Cl.: A61M 15/00

(54) **DRUG-DELIVERY DEVICE**
VORRICHTUNG ZUR ARZNEIMITTELVERABREICHUNG
DISPOSITIF D'ADMINISTRATION DE MÉDICAMENT

(30) Priority: 06.12.2022 GB 202218332
(43) Date of publication of application: 02.07.2025
(73) Proprietor: CAMBRIDGE HEALTHCARE INNOVATIONS LIMITED, Cambridge Cambridgeshire CB4 0WG (GB)
(72) Inventor: HARRIS, David, St Edmunds Suffolk IP29 5AX (GB); ISAACS, Warren, St Edmunds Suffolk IP29 5AX (GB); GREENWOOD, Jamie, St Edmunds Suffolk IP29 5AX (GB)
(74) Representative: Reddie & Grose LLP
(86) International application number: PCT/GB2023/053150
(87) International publication number: WO 2024/121559

(56) References cited:
- EP-B1- 1 296 732
- WO-A1-96/09085
- US-A1- 2007 074 721
- US-A1- 2017 326 312
- US-A1- 2018 207 374

## Description

### Field of Invention

The invention relates to a drug-delivery device for deagglomerating a dry powdered medicament comprising an active pharmaceutical ingredient (API) and a carrier.

### Background of Invention

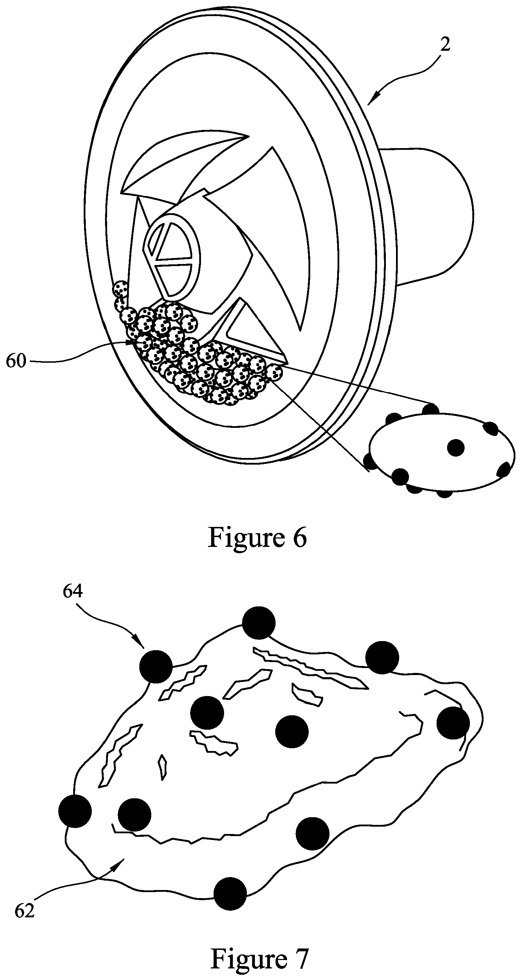

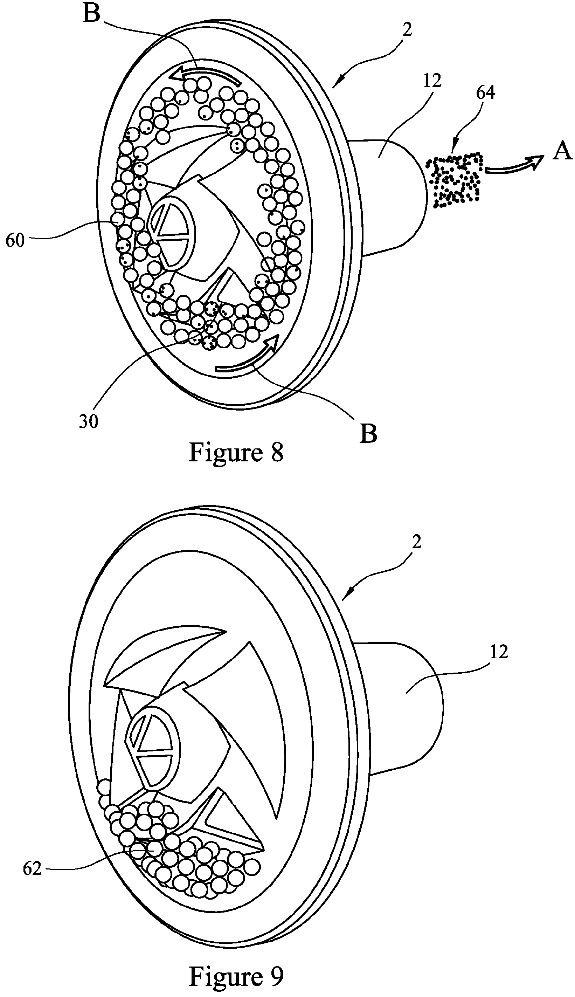

A dry powdered medicament for inhalation typically comprises an active pharmaceutical ingredient (API) in the form of a powder, mixed with a carrier powder such as lactose powder. The particle size of the API powder is very small, much smaller than the particle size of the carrier, and the total mass of API in a dose is typically very small. During processing, therefore, the very small mass of API is often blended with a much larger mass of the carrier powder. The API particles adhere to the carrier particles so that the combined medicament is easier to handle during packaging. Figure 7 is a schematic illustration of smaller API particles 64 adhering to a larger carrier particle 62.

However, if a dose of this medicament is administered using a conventional inhaler, a problem arises because the API remains adhered to the carrier particles and both the API and carrier particles are inhaled. The carrier particles are inert and so their inhalation causes no health problem, but the larger mass of the carrier particles prevents them from following the inhaled air-flow into a user's lungs where the API is intended to be delivered. The carrier particles with the API adhering to them are too massive to be carried effectively by the inhaled air-flow around corners in the user's throat (particularly at the back of the mouth where the airflow turns from the user's mouth into the user's windpipe). The particles of the medicament therefore tend to deposit in the user's throat and mouth before they reach their intended target in the lungs.

One solution to this problem may be not to combine the API with carrier particles. In that case, the user can inhale only the smaller API particles, and these smaller particles may be carried more effectively by the user's inhaled airflow and delivered directly the lungs. This may advantageously enhance the therapeutic effect of the API particles. However, for ease of handling the small API particles during manufacture and packaging, it is desirable to blend them with carrier particles.

When carrier particles are used, to solve the problem of a user inhaling a dose of medicament while the API is still adhering to the carrier particles, it is desirable to deagglomerate the medicament before inhalation to separate the API from the carrier so that the API is inhaled by itself. The smaller API particles can then be more effectively carried by the inhaled air flow into the user's lungs.

There are a number of constraints in designing an inhaler for deagglomerating an API powder from a carrier powder, including the fact that a user inhaling through a drug-delivery device can only generate a limited amount of energy. Inhalers which use only the energy in the inhaled air-flow may be termed passive inhalers. In passive inhalers, the energy available to deagglomerate the medicament is limited. In other inhalers, termed active inhalers, an additional source of energy such as a pressurised gas may be used to improve deagglomeration, but only at the cost of a much more complex drug-delivery device.

The problem of deagglomerating an API from a carrier is becoming more acute because there is increasing interest in large-dose medicaments for inhalation. This makes the problem of deagglomerating or classifying the medicament to separate the API from the carrier even more difficult, particularly in a passive inhaler. In a passive inhaler, the increased mass of medicament must be deagglomerated using the same inhalation energy available from a user.

An aim of the disclosure is to address the problem of efficient deagglomeration, and to enable efficient and effective deagglomeration and classification even for larger dose sizes, preferably using a passive inhaler.

Another aspect of the provision of dry-powder medicaments is the packaging of the medicaments. One simple and effective packaging method is blister packaging, in which a medicament is stored in a blister pocket sealed by a rupturable blister seal or lid, such as a blister foil. In the prior art, inhalers have been developed to allow inhalation of medicaments stored in blisters. Typically, in such an inhaler the lid of a blister may be pierced by a portion of an inhaler device and the entire dose of medicament removed from the blister for inhalation. Two examples of such inhalers are as follows.

Patent publication US 8,297,278 (Vectura Delivery Devices Ltd) describes an inhaler for delivering a dose of a dry powdered medicament. Each dose of the medicament is stored in a package, such as a blister, having a pierceable lid. The inhaler comprises a drug outlet tube terminating with a primary piercing element for piercing an outlet opening in the lid, and a secondary piercing element for piercing peripheral air inlet openings in the lid. The inhaler is connected to a source of pressurised gas, such as a piston pump, which delivers air through a plenum chamber to the secondary piercings and into the package to scour the interior of the package so that all or substantially all of the dose is entrained in the gas and flows out of the package through the drug outlet tube. The drug outlet tube directs the dose into a vortex chamber within the inhaler, which is coupled to a mouthpiece of the inhaler, where it is aerosolised for inhalation by the user.

Patent publication US 8,607,787 (Manta Devices LLC) describes an inhaler for a liquid or powdered medicament in which the medicament is stored in and, in use, delivered from a torus-shaped chamber. An air inlet delivers air tangentially to the outer edge of the torus-shaped chamber, and an air outlet receives air tangentially from the inner edge of the torus-shaped chamber. Air circulating within the torus-shaped chamber thus entrains the medicament and delivers it through the outlet. The air flow from the outlet is then combined with air passing through another passageway, to meter the dose to a user. US8,607,787 notes that a satisfactory combination of dispersion, fluidisation and metering may enhance drug delivery by allowing substantially all of the drug to be delivered at a desired rate, which may increase safety and reduce cost and waste. In some embodiments, the device described in US 8,607,787 may be used with a medicament stored in a specially-designed blister, manufactured with a central internal piercer. When the medicament is to be removed from the blister, the blister is deformed into a torus shape for use with the inhaler. As the blister is deformed, the internal piercer pierces the blister foil and forms the air outlet.

There are disadvantages in the designs of these and other prior art inhalers. For example the carrier particles in a dry powdered medicament, such as lactose, need to have surfaces to which the API particles adhere. The carrier particles also have a tendency to adhere to other materials. Therefore in inhalers which remove a medicament from a package such as a blister, there is a tendency for some of the carrier particles, still carrying API particles, to adhere to internal surfaces of the inhaler rather than being inhaled by the user. This causes several problems. One is that these particles may be dislodged when a subsequent dose of medicament is inhaled, meaning that the later dose may be larger than prescribed. Another is that the API particles are pharmaceutically active and so it is disadvantageous for such particles to remain in the environment after removal from their packaging. It would be better to avoid carrier particles, carrying an API, sticking to the internal surfaces of an inhaler.

In addition to improving deagglomeration of a powdered medicament, another object of the invention is to improve the safety of operation of an inhaler, by preventing any medicament which may not be delivered to a user from adhering to internal surfaces of the inhaler. The following documents are also known in the art, namely US 2007/074721 A1, EP 1 296 732 B1, US 2018/207374 A1, WO 96/09085 A1, and US 2017/326312 A1.

### Summary of Invention

The invention provides a drug-delivery device, an inhaler apparatus, and a blister for deagglomerating a powder as defined in the appended independent claims, to which reference should now be made. Preferred or advantageous features of the invention are set out in dependent subclaims.

In a preferred embodiment, the disclosure may thus provide a drug-delivery device for deagglomerating and/or classifying a powdered medicament comprising an active pharmaceutical ingredient (API) and a carrier. The device comprises an inhaler apparatus and a blister, which combine to form an apparatus for deagglomerating the medicament for inhalation, by separating (or classifying) the API from the carrier. The blister contains the medicament, and comprises a circular blister pocket sealed by a pierceable or rupturable seal, such as a foil seal. The inhaler apparatus comprises; an air-outlet piercer for piercing the seal to form an air outlet, through which air can be drawn towards an inhaler mouthpiece for delivering a flow of deagglomerated medicament to a user; and two or more (preferably three, four or five) air-inlet piercers, each for piercing the seal to form an air inlet for directing air into the blister when air is drawn through the outlet.

When the piercers pierce the seal, the air outlet is advantageously axially positioned in the circular blister, and each inlet is positioned between the air outlet and an edge of the blister. Each inlet is advantageously shaped so that, in use, air drawn through the inlet enters the blister in a tangential direction to create a cyclonic airflow within the blister, and so that an upstream end of each air inlet presents a streamlined profile to the cyclonic airflow within the blister. When the seal is pierced, one or more of the air-inlet piercers advantageously extend(s) into the blister by less than the distance which the air-outlet piercer extends into the blister. The circular blister pocket has a maximum lateral dimension and one or more of the inlets is/are advantageously spaced from an outer edge of the blister by more than 10% of the maximum lateral dimension of the blister, and from a central axis of the blister by more than 10% of the maximum lateral dimension of the blister.

In use, the inhaler apparatus is initially engaged with the blister so that the piercers pierce the seal. When air is then drawn from the blister through the air outlet, in an air-outlet direction, the air inlets are positioned to direct the air flowing into the blister substantially tangentially to the air-outlet direction, so that a circulating, or cyclonic, airflow is created within the blister. The cyclonic airflow advantageously carries the medicament circumferentially within the blister, deagglomerating the API from the carrier as particles collide with each other and with walls of the blister. The cyclonic airflow also advantageously accelerates the medicament away from the air outlet, so that only smaller, lighter particles in the medicament can be drawn through the air outlet. The API particles are smaller and lighter than the carrier particles, and so the API is preferentially drawn through the outlet, classifying the medicament, for inhalation by a user.

In a particularly preferred embodiment the inventors understand that the operation of the drug delivery device is as follows. A powdered medicament comprising an active pharmaceutical ingredient (API) and a carrier powder is provided in a circular blister. An inhaler apparatus has an air outlet piercer for axially piercing the blister, and three, four or five air-inlet piercers, each for piercing an inlet opening into the blister and for directing air into the blister when air is drawn through the outlet by a user inhaling through the inhaler apparatus. The inlets direct a tangential airflow into the blister to set up a cyclonic airflow which deagglomerates the powdered medicament so that the API is separated from the carrier particles and is carried, in the airflow, through the outlet piercer for inhalation. In use, an upstream end of each air inlet presents a streamlined profile to the cyclonic airflow within the blister, one or more of the air-inlet piercers extend(s) into the blister by less than the distance which the air-outlet piercer extends into the blister, and one or more of the inlets is/are spaced from an outer edge of the blister by more than 10% of the diameter of the blister, and from a central axis of the blister by more than 10% of the diameter of the blister. In use, the cyclonic airflow within the blister creates a low pressure core in the region of the air outlet piercer. The circulating cyclonic airflow thus carries the powdered medicament and accelerates it radially outwards within the blister, such that flow of the smaller, lighter API particles towards the low pressure core and through the air outlet is favoured relative to flow of the larger, heavier carrier powder particles through the outlet.

In a preferred embodiment, substantially all of the API particles may be withdrawn from the blister with the outlet airflow, while substantially all of the carrier particles remain within the blister, ready for disposal with the used blister.

In use of embodiments of the invention, a user first engages the inhaler apparatus with a blister containing powdered medicament, by inserting the air-outlet piercer and the air-inlet piercers through the seal. The air-outlet piercer and the air-inlet piercers are preferably formed as part of a single component, for example a moulded plastic component. In a particularly preferred embodiment, the inhaler apparatus further comprises a flat surface from which the piercers extend, so that the flat surface abuts an outer surface of the seal after the piercers have pierced the seal. This may advantageously control or determine the depths to which the outlet and inlet piercers enter the blister. The inhaler apparatus may additionally engage with another portion of the blister to ensure that the blister is correctly positioned relative to, or is in registration with, the piercers when the seal is pierced. For example, the inhaler apparatus and/or the blister may comprise a visual indicator for aiding rotational or lateral alignment of the inhaler apparatus with the blister, and/or the blister and/or the inhaler apparatus may comprise co-operating mechanical shapes or keys to ensure alignment. The user may then inhale through the inhaler apparatus, drawing air through the air-outlet piercer. This, in turn, draws air through the air-inlet piercers and creates a cyclonic airflow within the blister, accelerating the medicament radially outwardly and carrying the medicament circumferentially around the blister. The particles of the API are smaller and lighter than the carrier particles (which might, for example, be lactose particles) and therefore the API particles are preferentially drawn out of the blister through the air-outlet piercer for inhalation by the user.

The inventors have found that embodiments of the disclosure deagglomerate and classify the medicament very effectively. Advantageously, after operation of embodiments of the invention, more than 70%, 80% or 90% of the carrier particles are retained in the blister while more than 70%, 80% or 90% of the API particles have been deagglomerated from the carrier particles, removed from the blister and inhaled. The inventors have found that devices embodying the disclosure are extremely effective classifiers to separate API particles from carrier particles.

It may be noted that in preferred embodiments of the invention, the carrier particles which are classified, or separated, from the API particles during inhalation may advantageously be retained in the blister. Any API particles which are not delivered to the user are also retained in the blister. This simplifies operation of the drug-delivery device, because no portion of the medicament may be retained in the inhaler apparatus, or is lost into the environment, and any carrier particles or API not inhaled by the user are conveniently and safely disposed of by disposing of the used blister.

Embodiments of the disclosure may advantageously be used for high-payload drug-delivery devices, in which a large quantity of powdered medicament (for example 50 mg and above) is contained within the blister. To deagglomerate, or classify, a larger medicament payload in particular, it is preferable to maximise the air flow rate through an inhaler for a given inhalation pressure exerted by a user. This may maximise the power (pressure drop multiplied by flow rate) available to deagglomerate and classify the medicament. To that end, all of the air inhaled by the user advantageously passes through the air inlets, the blister and the air outlet (although in some embodiments there may be a small air bypass to modify the sensation felt by the user inhaling through the device).

The drug-delivery device is preferably a high-resistance or ultra-high-resistance inhaler. This may advantageously allow a consistent airflow through the device to be achieved by users capable of applying different inspiratory efforts.

A high-resistance inhaler typically allows an air flow rate of less than about 60 I/min when a differential pressure of 4 kPa is applied across the inhaler, and an ultra-high resistance inhaler allows a flow rate of less than about 45 l/min at 4 kPa. In a preferred embodiment of the disclosure an air flow rate as low as 40 I/min or less may be achieved when a differential pressure of 4 kPa is applied. As well as allowing a consistent airflow through the device for different users, the low flow rate through the inhaler apparatus and the blister is partly a consequence of high viscous momentum transfer losses in the free vortex in the outer portion of the blister, during inhalation. These momentum transfer losses enhance classification of the API from the carrier within the blister, so that a greater proportion of the API is inhaled and a greater proportion of the carrier is retained in the blister after inhalation. In a particularly-preferred embodiment, the inventors have achieved very high classification levels of 94%.

Further preferred features of the disclosure will now be discussed.

Preferably, an edge of the blister pocket against which the seal is secured is circular. The cyclonic airflow may then circulate unimpeded within the blister, or blister pocket, advantageously maximising the speed of the airflow, and therefore maximising the acceleration applied to the medicament and the effectiveness of both deagglomeration and classification.

To create the desired circulating airflow within the blister, or blister pocket, when the air-inlet piercers pierce the seal, the inlets are positioned between the air outlet and an edge of the blister. The inventors have found that selecting the position of the air inlets affects the performance of embodiments of the disclosure in a surprising way.

In a cyclone separator, air inlets are typically positioned at the outer radial edge of a cyclone chamber, to maximise the speed and energy of the airflow within the cyclone. This is for the following reasons. The peak energy in the airflow in the cyclone is at the inlet radius. At radii smaller than the inlet radius, i.e. between the inlets and the central outlet of the cyclone, the circulating air tends to behave as a forced vortex with the airspeed at any point proportional to its radius of rotation. In principle, the airspeed falls to zero at the centre of the low pressure core of the cyclone. At radii greater than the inlet radius, i.e. between the inlets and an outer edge of the cyclone chamber, the circulating air tends to behave as a free vortex with the airspeed at any point inversely proportional to its radius of rotation, falling from a maximum at the inlet radius to zero at a boundary layer in contact with the outer edge of the cyclone chamber.

Therefore, in drug delivery devices embodying the disclosure, the skilled person would expect that positioning the air inlets at or close to an outer edge of the blister should maximise the speed and energy of the airflow in the blister, and so maximise the deagglomeration and classification effect of the cyclone within the blister. But the inventors have found that spacing the air inlets away from the outer edge of the blister provides surprisingly improved performance. In the inventors' experiments, spacing the air inlets away from the outer edge of the blister enhances deagglomeration and classification of the medicament despite the theoretical expectation that this should reduce the speed of the airflow within the blister. The inventors' understanding of their experimental observations is that, because the blister contains a relatively large volume of powdered medicament, there is advantage in providing sufficient space in the blister outside the radius of the inlets to allow the powder to circulate freely within the blister. This is illustrated in Figures 6, 8 and 9. The momentum of the powder, and impacts between the moving powder and the outer wall of the cyclone chamber (i.e. the blister), disrupt the boundary layer at the outer wall of the blister and allow a higher airspeed, and rate of rotation of the air (and powder) within the blister, than would occur in the absence of the powder. Without the powder circulating in the blister, the airflow outside the radius of the inlets would form a free vortex as in a conventional cyclone as described in the paragraph above, and the airspeed at the boundary layer at the outer edge of the blister would fall to zero.

Putting this into practice, therefore, if the blister has a maximum lateral dimension (i.e. a diameter for a circular blister) then as noted above one or more of the inlets (measured at a centre of each inlet) is/are spaced from an outer edge of the blister by more than 10% of the maximum lateral dimension of the blister. In preferred embodiments the spacing from the outer edge of the blister may be greater, such as more than 15% or 20% of the maximum lateral dimension of the blister.

Particularly preferably, all of the inlets (measured from the centre of each inlet) may be spaced from the outer edge of the blister by more than 10% of the maximum lateral dimension of the blister, and preferably by more than 15% or 20% of the maximum lateral dimension of the blister.

In addition, the inventors have found that performance of the deagglomerator may be improved by spacing the air inlets sufficiently far from the air outlet, or from the centre of the blister. Thus, if a blister has a maximum lateral dimension (i.e. a diameter for a circular blister) then as noted above one or more of the inlets (measured at the centre of each inlet) is/are spaced from a central axis of the blister by more than 10% of the maximum lateral dimension of the blister. In preferred embodiments this spacing may be greater, such as more than 15% or 20% of the maximum lateral dimension of the blister.

Particularly preferably, all of the inlets may be spaced from the central axis of the blister by more than 10% of the maximum lateral dimension of the blister, and preferably by more than 15% or 20% of the maximum lateral dimension of the blister.

In embodiments of the disclosure, it is preferable but not essential that all of the air inlets are at the same distance from the air outlet and/or are at the same distance from the outer edge of the blister.

As noted above an upstream end of each air inlet presents a streamlined profile to the cyclonic airflow within the blister. In a preferred embodiment the upstream end of each air inlet may comprise a streamlined profile, for example in the form of a streamlined shroud, to reduce resistance to the cyclonic airflow. Advantageously, the streamlined profile or shroud may be positioned at an angle of less than 40 degrees, or less than 30 degrees or 20 degrees, to the circumferential direction of the cyclonic airflow, so as to reduce air resistance.

In order to achieve a fast and stable cyclonic airflow in the blister, the inlets are preferably narrow in width, for example measured along a radial axis of the blister. If the inlets are relatively narrow, the space in the blister outside the inlets, between an outer edge or wall of each inlet and an outer edge or wall of the blister, is advantageously large and provides a free space for rapid cyclonic airflow. Similarly if the inlets are relatively narrow, the space in the blister between the outlet piercer and the inlets, between an inner edge or wall of each inlet and the outlet piercer, is advantageously large and provides free space for the cyclonic airflow and to set up a low pressure cyclonic core in the region of the outlet piercer, for good classification of the API from the carrier particles.

In a preferred embodiment, the width of one or more inlets, or of all of the inlets, is less than 20% of the maximum lateral dimension of the blister, and is preferably less than 15% or 10% of the maximum lateral dimension of the blister.

As mentioned above, in a preferred embodiment the drug-delivery device is a high-resistance device or ultra-high-resistance device, meaning that its performance is preferably relatively independent of the inspiratory effort of a user, and so that effective classification is achieved. The dimensions of the outlet and/or the inlets are preferably selected to achieve this.

According to the invention, the inhaler apparatus comprises between 3 and 5 air-inlet piercers, and particularly preferably 4 air-inlet piercers. The inventors have found that the speed of cyclonic airflow through the blister may be increased by reducing the number of air-inlet piercers, but that increasing the number of air-inlet piercers, and preferably spacing them around the air-outlet piercer, improves stability of the cyclonic airflow within the blister, leading to more efficient and predictable deagglomeration and classification of the medicament.

Further preferred features of the invention relate to the design of the air-outlet piercer and the air-inlet piercers.

The air-outlet piercer has a lateral dimension, and when the seal is pierced, at least a peripheral wall of the air-outlet piercer preferably extends into the blister by a distance greater than half of that lateral dimension. For example, the air-outlet piercer may be in the form of a tube having a peripheral wall, and may have a sharpened lower end, or a shaped lower end, for piercing or cutting the seal. The tube may have a circular cross-section, in which case its lateral dimension may be its diameter. The fact that the peripheral wall of the air-outlet piercer extends by a sufficient distance through the seal, into the blister, may have the effect that no portion of the seal displaced by the air-outlet piercer impedes airflow through the outlet, or occludes the outlet.

The piercing end of the air-outlet piercer may comprise a central, or axial, point or cutting element, protruding beyond other portions of the air-outlet piercer so as to contact the seal before other portions of the piercer. This may ensure that the seal ruptures at the centre of the piercer. The piercing end of the air-outlet piercer may further comprise a plurality of blades or edges extending radially from the central point, to cut the seal during piercing in a progressive, controlled and predictable way. This may further ensure that no portion of the seal displaced by the air-outlet piercer impedes airflow through the outlet.

In a preferred embodiment, the depth of the blister, or the blister pocket, is greater than 1.2 or 1.5 or 2 times the distance which the air-outlet piercer extends into the blister. Particularly preferably, the depth of the blister, or the blister pocket, is greater than 1.2 or 1.5 or 2 times the distance which the peripheral wall of the air-outlet piercer extends into the blister. The depth of the blister may even be greater than 3 times the distance which the air-outlet piercer, or the peripheral wall of the air-outlet piercer, extends into the blister. This dimension ensures that there is sufficient clearance between the air-outlet piercer, or the air outlet, and the base of the blister pocket to allow a rapid cyclonic airflow within the blister pocket.

In addition, when the seal is pierced, one or more of the air-inlet piercers preferably extend(s) into the blister by less than the distance which the air-outlet piercer extends into the blister. This may advantageously allow the air-outlet piercer to contact the seal first, followed by the air-inlet piercers contacting the seal. This may enhance progressive and predictable cutting of the seal during piercing. The purpose of the air inlets is to direct inflowing air in a tangential direction within the blister. To achieve this, all of the air inlets are oriented in the same direction, clockwise or anticlockwise.

One or more of the air-inlet piercers (and preferably all of the air-inlet piercers) preferably extend(s) into the blister by approximately the same distance as the peripheral wall of the outlet piercer extends into the blister, for example by between 0.7 and 1.3, or between 0.9 and 1.1 times the distance that the peripheral wall of the outlet piercer extends into the blister.

As a cyclonic or circulating airflow is set up within the blister, it is preferred that any resistance to the cyclonic airflow is minimised. The blister pocket and an inner surface of the blister seal define the space within which the cyclonic flow circulates, and the air-outlet piercer and the air-inlet piercers protrude through the seal into this space. The air-outlet piercer is centrally, or axially, located with respect to the blister, and so has relatively little effect on the cyclonic flow which moves fastest at the outer periphery of the blister. However, the end of the air-outlet piercer through which air is drawn out of the blister is preferably located in a low-pressure core of the cyclonic flow, to ensure that the API is effectively classified from the carrier particles.

By contrast, the air-inlet piercers are spaced from the air-outlet piercer, between the air-outlet piercer and the outer periphery of the blister, and so potentially hinder the cyclonic flow. Therefore, the-inlet piercers are advantageously shaped at their upstream ends to reduce any resistance they present to the cyclonic flow.

In a preferred embodiment, one or more of the air-inlet piercers comprises two walls meeting or converging at a piercing edge which is shaped to progressively pierce or cut the seal as the piercers moved through the seal. In other words, a radially inner surface and a radially outer surface of each air-inlet piercer converge into the piercing edge.

The two walls are shaped so that the air-inlet piercer tapers outwardly, or widens, from its upstream end to its downstream end at which an air-inlet opening is defined between ends of the walls. Consequently, in use, air flowing within the air-inlet piercer, between the walls, enters the blister through the air-inlet opening in a substantially tangential direction to create the desired cyclonic flow. In other words, an inner surface of the air-inlet piercer is shaped to receive an airflow from outside the blister and to direct it within the blister so as to create the cyclonic flow.

Advantageously, the inner surface of the air-inlet piercer and the air-inlet opening through which incoming air enters the blister are shaped so as to minimise air resistance. For example, the shape of the air-inlet opening should be a low-aspect-ratio shape. The air-inlet opening is preferably oriented on a plane intersecting an axis of the air outlet, so that air leaves the air-inlet opening in a tangential direction. In that case, a maximum dimension of the air-inlet opening in the radial direction should be approximately equal to, or within 70% to 130% of, its maximum dimension in the axial direction. For example, where the air-inlet opening is defined between the ends of two walls which meet at a piercing edge, the air-inlet opening may advantageously be substantially an equilateral triangle.

At the same time, the tapered shape of the air-inlet piercer at its upstream end presents a streamlined profile to the cyclonic airflow, minimising air resistance. The outer surfaces of the walls or the sides of the air-inlet piercer may be substantially flat, or straight, and oriented substantially tangentially within the blister. In a preferred embodiment, however, to further reduce air resistance for the cyclonic flow, the or each air-inlet piercer may have a radially-inner surface with a curved concave profile and radially-outer surface with a curved convex profile, in a circumferential direction within the blister, shaped to match the radii at which each side of the air-inlet piercer is positioned within the blister.

At the same time, the or each air-inlet piercer may have a radially-inner surface with a convex profile and radially-outer surface with a concave profile, in a direction parallel to the axis of the blister. This shaped air-inlet piercer may increase air resistance but may advantageously direct medicament which collides with the air-inlet piercer outwardly within the blister, enhancing deagglomeration.

Preferably, the plurality of air-inlet piercers may be arranged in a circular pattern surrounding the air-outlet piercer, at a predetermined radius from an axis of the air outlet. Particularly preferably, the air-inlet piercers are arranged symmetrically around the outlet, at equal spacings from each other. This may enhance the stability of the cyclonic flow within the blister.

In practice, the arrangement of the air outlet, air inlets and the interior surface of the blister are such that, in use, the flow of air within the blister is turbulent rather than laminar. This may encourage a more stable cyclonic flow. Further, the air inlets are preferably configured to promote a free vortex flow surrounding the air inlets.

An air-inlet piercer is preferably shaped so as to cut the seal in a predictable manner, without any portion of the cut seal impeding airflow out of the-inlet opening. To achieve this, the seal-cutting edge between the walls of the air-inlet piercer may terminate at a point adjacent to the air-inlet opening, so that penetration of the piercer through the seal forms two substantially triangular portions of the seal lying alongside the walls of the piercer, which abut each other at their respective corners adjacent to the upstream end of the piercer. The triangular portions of the seal form smooth surfaces over which the cyclonic airflow can easily pass. It should be noted that the two triangular seal portions formed by the penetration of the piercer through the seal have their apices adjacent to the air-inlet opening, which is the highest point of the piercer, and converge at the upstream end of the piercer, which is the lowest point of the piercer. In a preferred embodiment, the upstream end of the piercer may be shaped so that it does not cut the seal at the upstream ends of the two triangular seal portions, but deforms the seal to present a smoothly-curved seal portion to the oncoming cyclonic airflow. This may further reduce the air resistance presented by the air inlets to the cyclonic airflow.

To minimise air resistance, the piercing edge of the air-inlet piercer is advantageously at a low angle to the tangential direction of the cyclonic airflow. Preferably, the piercing edge is at an angle of less than 20°, or 30°, or 40° to the tangential direction.

At the same time, the air outlet of the air-inlet piercer is preferably oriented at a large angle to the tangential direction, such as between 70° or 80° and 100°, and is preferably perpendicular to the tangential direction. In this way, the air flowing out of the air inlet induces a maximum rate of cyclonic airflow.

The walls of the air-inlet piercer, meeting at the piercing edge, may therefore form a streamlined shroud behind the air outlet, with the walls and the piercing edge at a lower angle to the tangential direction of the cyclonic airflow than the angle between the air inlet and the tangential direction of the cyclonic airflow. The angle between the piercing edge and the tangential direction may be less than 50%, and preferably less than 40% or 30% of the angle between the air inlet and the tangential direction.

The end of the air-inlet piercer defining the air-inlet opening is preferably arranged at an angle to the seal, such as substantially perpendicular to the seal, so that no portion of cut seal overhangs or occludes the air-inlet opening.

In summary, each air-inlet piercer is preferably shaped so that, in use, air drawn through the air inlets enters the blister in a tangential direction, and so that the upstream ends of the air inlets present a streamlined profile to the cyclonic airflow within the blister.

References to the air inlets directing air in a tangential direction may include directing air in a direction which is not precisely tangential but which contains a significant tangential component, as required to generate the desired cyclonic airflow.

The size of each air inlet needs to be considered, as well as its shape. For example, the number and sizes of the air inlets may be selected so that the inlets cumulatively provide less air resistance than the outlet. As also noted above, all of the air inhaled by a user preferably passes through the inlets, the blister and the outlet.

At the same time, the size of each air inlet may advantageously be minimised so that the inlets impede cyclonic flow within the blister as little as possible. Preferably, therefore, the cross-sectional area of each inlet, perpendicular to the direction of the cyclonic flow, is advantageously minimised, and the shape of the air-inlet piercer is streamlined to minimise aerodynamic drag.

The air-inlet piercers may not all be the same shape or size as each other. For example, one or more air-inlet piercers may have greater maximum height than one or more others, so that the piercers pierce the seal sequentially rather than simultaneously as the inhaler apparatus engages with the blister.

Air-inlet piercers may be positioned at different radial distances from the air-outlet piercer, and/or at different angles to the tangential cyclonic airflow, in order to introduce some variability in the cyclonic flow as it circulates. This may enhance deagglomeration, if a particular medicament requires greater deagglomeration, for example if an API adheres strongly to its carrier. By contrast, placing all the air-inlet piercers at the same radius within the blister may enhance classification by maximising radial acceleration within the cyclonic flow.

Preferably, the blister pocket is shaped to enhance cyclonic flow within the blister. Typically, the blister may comprise a blister pocket sealed with a flat, or planar, seal such as a foil seal. At the outer edge of the blister, an angle is therefore defined between a peripheral wall of the blister pocket and the seal. During inhalation through the drug-delivery device, the medicament is accelerated outwardly within the blister, carried by the cyclonic flow. In a preferred embodiment, therefore, the angle between the peripheral wall of the blister pocket and the seal is advantageously as large as possible, for example being greater than 30° or 40°, and up to 50°, 60°, 70°, 80° or 90°.

At the same time, the blister pocket should be deep enough to accommodate the air-outlet piercer and the air-inlet piercers. Blister pockets are conventionally moulded by deforming flat sheets of material. This may be carried out by methods including deep drawing and cold forming. Any method may be used to form blisters embodying the invention, but the blister pocket should advantageously have a smooth inner surface to optimise the cyclonic flow. Deep drawing tends to introduce wrinkles into the material of the blister pocket and so cold forming may be preferred.

One way to achieve a sufficiently deep blister pocket with a relatively large angle between the peripheral wall of the blister pocket and the seal is to use a blister pocket with a cross-section shaped as a sector of a circle, such as a hemisphere. This shape may also maximise the volume of the blister pocket. However, when a circular-section blister pocket is formed the strain in the material is greatest at the outer edge of the blister pocket. The inventors have found that moulding a blister pocket with a parabolic cross section may enable formation of a blister pocket with lower strain at its outer edge than a circular-section blister pocket of the same depth (so as to accommodate an air-outlet piercer of a given size). Use of a parabolic cross section may be particularly advantageous if a blister pocket is formed from a sheet of material comprising an aluminium vapour barrier (such as a plastic-aluminium-plastic laminate), which may be damaged by straining the material beyond a certain threshold, typically 32% strain. When such a material is used, a deeper blister pocket may advantageously be moulded using a parabolic shape rather than a circular, or spherical, shape.

It should be noted that, advantageously, embodiments of the invention may not require any structure or features within the blister, so that the blister only comprises, or consists of the blister pocket and the seal, with the medicament sealed inside. This may facilitate manufacture of the blister. In addition, no deformation of the blister or modification of the blister is required during use of the drug-delivery device, except for the piercing of the seal. The blister may therefore be fabricated from conventional materials, selected for optimum compatibility with the medicament.

In a further aspect, the invention may advantageously provide an inhaler apparatus as described, for engaging with a blister containing a medicament.

In another further aspect, the disclosure may advantageously provide a blister suitable for engaging with an inhaler apparatus as described.

In different embodiments of the invention, the inhaler apparatus may engage with blister in different ways. For example, the inhaler apparatus may move towards the seal in a perpendicular direction or it may be hinged so that it pivots into engagement with the blister.

In a still further aspect, the disclosure may also advantageously provide a method for deagglomerating and/or classifying a powdered medicament comprising an active pharmaceutical ingredient (API) and a carrier, in which the medicament is contained in a blister, comprising a blister pocket sealed by a pierceable seal. The blister is engageable with an inhaler apparatus which comprises an air-outlet piercer for piercing an air outlet through the seal, through which air can be drawn towards an inhaler mouthpiece, and a plurality of air-inlet piercers, each for piercing an inlet opening through the seal for directing air into the blister when air is drawn through the outlet.

The method may then comprise the steps of bringing the inhaler apparatus into engagement with the blister to pierce the seal with the air-outlet and air-inlet piercers, and a user inhaling through an inhaler mouthpiece coupled to the outlet, so that the blister forms a cyclone for deagglomerating and classifying the medicament. Air is drawn from the blister through the outlet opening in an air-outlet direction, and the air inlets surround the air outlet and direct the air substantially tangentially to the air-outlet direction to form a cyclonic airflow within the blister.

### Embodiments of the Invention

Specific embodiments of the invention will now be described by way of example, with reference to the accompanying drawings, in which;
Figure 1 is a three-quarter view of a drug-delivery apparatus according to a first embodiment of the invention (with a blister shown cut away for clarity);
Figure 2 is a shaded version of Figure 1;
Figure 3 is a side view of the drug-delivery device of Figure 1;
Figure 4 is a three-quarter view of a blister pocket according to the embodiment of Figure 1;
Figure 5 is an axial section of the blister according to the embodiment of Figure 1;
Figure 6 illustrates the drug-delivery device of Figure 1 before use;
Figure 7 is a schematic illustration of a carrier particle blended with an API within a powdered medicament;
Figure 8 is a schematic diagram of the drug-delivery device of Figure 1 during inhalation; and
Figure 9 illustrates the drug-delivery device of Figure 1 after use.

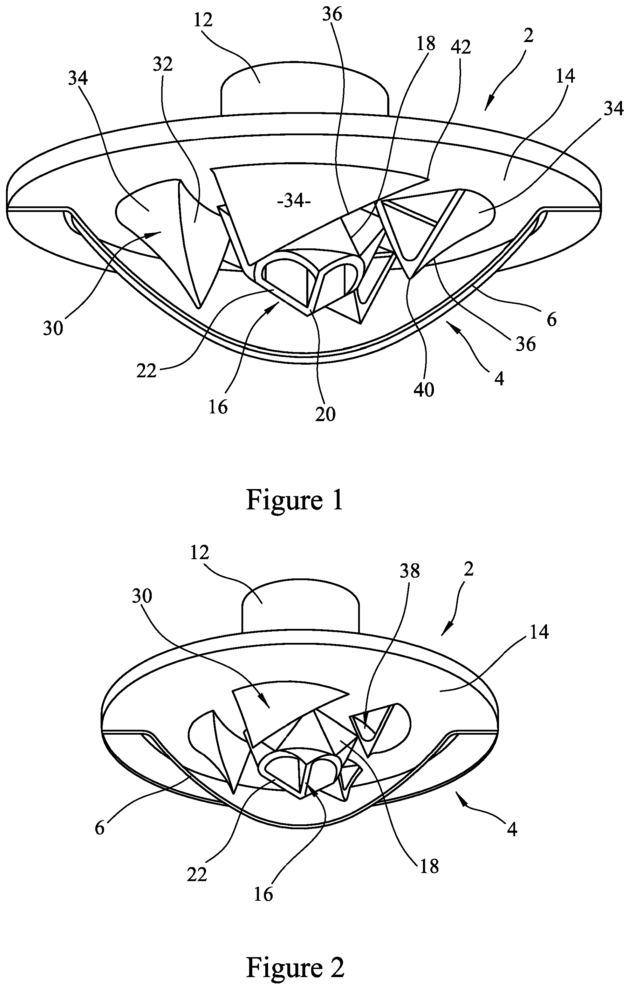

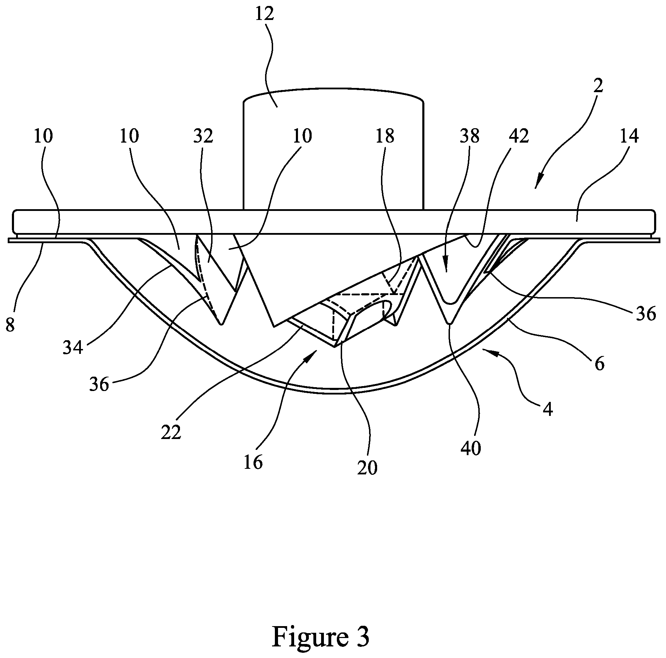

Figures 1, 2 and 3 illustrate a drug-delivery device according to a first embodiment of the invention, comprising an inhaler apparatus 2 engaged with a blister 4 (in the drawing, part of the blister is cut away and the blister seal and the medicament contained in the blister are not shown, to show the structure of the inhaler apparatus more clearly).

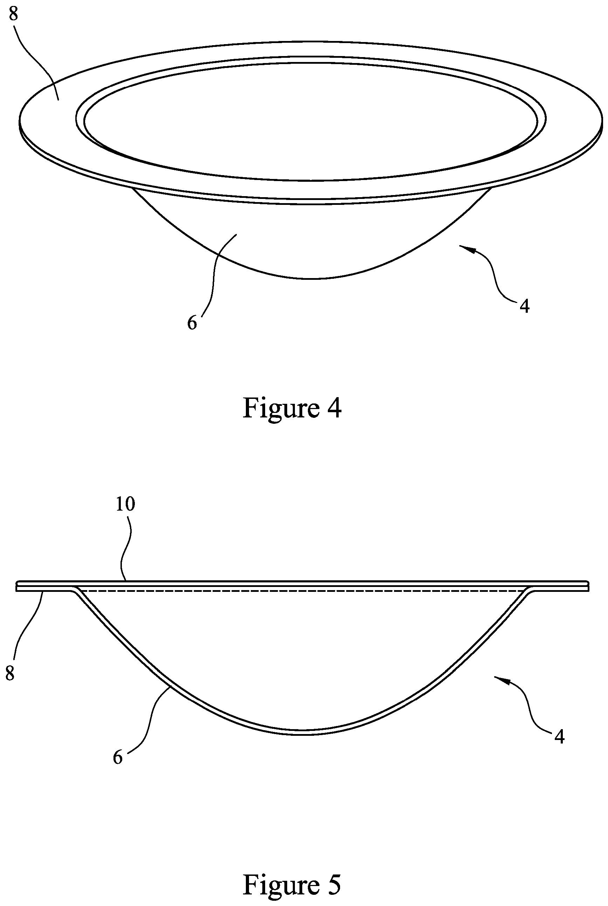

Figures 4 and 5 illustrate the blister 4, comprising a blister pocket 6 surrounded by a flange 8 and sealed by a blister seal 10. The outer edge of the blister pocket is circular, with a diameter between 22 mm and 23 mm, and an internal volume of 1400 mm³. The cross-sectional shape of the blister pocket is parabolic. The angle between the flange 8 and the outer edge of the blister pocket is moulded so that the strain in the material when the blister pocket is formed is less than 32%, meaning that the blister pocket can be cold formed from a laminate including a moisture-resistant aluminium layer.

As shown in Figures 1, 2 and 3, the inhaler apparatus comprises an air outlet 12 extending from a blister-engaging plate 14. From the plate 14, the air outlet 12 terminates at an air-outlet piercer 16. The piercer comprises a peripheral wall 18 terminating in a seal-cutting edge, and a cruciform-shaped central cutter terminating in a central piercing tip 20. Four raked cutting edges 22 extend from the central tip to the peripheral wall.

When this air-outlet piercer pierces the blister seal, it first penetrates or ruptures the seal at the piercing tip 20. As the piercer advances through the seal, the seal is cut by the four raked cutting edges to form four triangular seal portions or flaps between the cutting edges. As the piercer is pushed fully through the seal, the triangular seal portions abut an outer surface of the peripheral wall. The height of the peripheral wall 18 protruding from the blister-engaging plate 14 is equal to or slightly greater than the height of the triangular seal portions so that these seal portions do not occlude the air outlet.

Four air-inlet piercers 30 also extend from the blister-engaging plate 14, surrounding the air-outlet piercer. The air-inlet piercers are positioned at the same radius from the axis of the air-outlet piercer, and are as equal 90° angles around the-outlet piercer. The air-outlet piercer is positioned centrally within the blister, and the central points of the air-inlet piercers are positioned halfway between the axis of the air-outlet piercer (and of the blister) and the peripheral edge of the blister pocket.

Each air-inlet piercer comprises two generally triangular walls 32, 34 converging at a piercing edge 36. As a downstream end of the piercer, a triangular air inlet 38 is defined between ends of the walls and an edge of the blister-engaging plate 14. The air inlet is at a large angle to the plate 14, of about 80°. An end of the piercing edge 36 adjacent to the air inlet 38 forms a piercing tip 40, and the piercing edge extends upstream away from the piercing tip 40 until it meets the blister-engaging plate 14 at its opposite end 42.

The bases of the walls of the air-inlet piercers, where they meet the blister-engaging plate, are curved, approximately along circumferential directions around the axis of the air outlet.

When the air-inlet piercers pierce the blister seal, they first contact the seal at the piercing tips 40. As each piercer advances through the seal, the piercing edge and the ends of the triangular walls adjacent to the air inlet cut through the seal, raising two triangular portions of the seal which abut the triangular walls of the piercer. Advantageously, no portion of the seal can then occlude the air inlet.

Figure 6 illustrates the drug-delivery device of Figures 1, 2 and 3 loaded with a powdered medicament 60 before use. In this drawing, the blister pocket is shown in ghost so that the medicament can be seen. Figure 5 illustrates a particle of the medicament in more detail. This comprises a lactose carrier particle 62 with a plurality of much smaller API particles 64 and adhering to it. When a medicament like this is inhaled, an object of the invention is to deagglomerate the medicament by separating as many API particles from the carrier particles as possible and to classify the medicament so that as many API particles as possible, and as few carrier particles as possible, are entrained into the air outlet for inhalation. The inventors' understanding is that the API may be separated from the carrier particles by mechanical interactions between one particle and another, and between the particles and the walls of the drug-delivery device, and that cyclonic action of the airflow within the blister during inhalation preferentially allows the smaller API particles to enter the centrally-positioned air outlet.

Figure 8 illustrates the drug-delivery device in use. A user inhales through a mouthpiece coupled to the air outlet 12. This inhalation draws air A through the outlet and, in turn, through the air-inlet piercers 30. The tangential, or circumferential, orientation of the airflow through the air inlets drives a rapid circumferential, cyclonic airflow B within the blister, which carries the medicament 60 with it. As the medicament circulates, medicament particles collide and the API is separated from the carrier. The cyclonic airflow accelerates the particles radially outwardly so that only the smallest, lightest particles can be carried by the airflow through the air outlet. These are the API particles 64 which are preferentially carried through the air outlet and inhaled.

Figure 9 shows the drug-delivery device after use, containing primarily carrier particles 62. The blister may now be disengaged from the inhaler apparatus and disposed of. The inhaler apparatus may be reused, by being engaged with a fresh blister containing medicament.

## Claims

1. A drug-delivery device comprising an inhaler apparatus (2) and a blister (4), for deagglomerating, within the blister, a powdered medicament (60) comprising an active pharmaceutical ingredient (API) (64) and carrier particles (62) blended with the API, in which;
the blister contains the medicament, and comprises a circular blister pocket (6) sealed by a pierceable seal (10); and
the inhaler apparatus comprises;
an air-outlet piercer (16) for piercing an air outlet (12) through the seal, through which air can be drawn towards an inhaler mouthpiece, the air outlet being axially positioned in the blister; and
three, four or five air-inlet piercers (30), each for piercing an inlet opening (38) through the seal for directing air into the blister when air is drawn through the outlet, each inlet being positioned between the air outlet and an edge of the blister and shaped so that, in use, air drawn through the inlet enters the blister in a tangential direction to create a cyclonic airflow within the blister, and so that an upstream end of each air inlet presents a streamlined profile to the cyclonic airflow within the blister;
in which when the seal is pierced, one or more of the air-inlet piercers extend(s) into the blister by less than the distance which the air-outlet piercer extends into the blister;
in which the blister pocket (6) has a parabolic cross section, in which the circular blister pocket has a maximum lateral dimension and one or more of the inlets is/are spaced from an outer edge of the blister by more than 10% of the maximum lateral dimension of the blister, and from a central axis of the blister by more than 10% of the maximum lateral dimension of the blister; and in which, in use, the cyclonic airflow within the blister carries the powdered medicament and accelerates the powdered medicament radially outwards within the blister, such that flow of the API through the air outlet is favoured relative to flow of the carrier through the outlet.

2. A drug-delivery device according to claim 1, in which one or more of the inlets (38) is/are spaced from the outer edge of the blister (4) by more than 15% or 20% of the maximum lateral dimension of the blister.

3. A drug-delivery device according to claim 1 or 2, in which all of the inlets (38) are spaced from the outer edge of the blister (4) by more than 10% of the maximum lateral dimension of the blister, and preferably by more than 15% or 20% of the maximum lateral dimension of the blister.

4. A drug-delivery device according to claim 1, 2 or 3, in which one or more of the inlets (38) is/are spaced from the central axis of the blister (4) by more than 15% or 20% of the maximum lateral dimension of the blister.

5. A drug-delivery device according to any preceding claim, in which all of the inlets (38) are spaced from the central axis of the blister (4) by more than 10% of the maximum lateral dimension of the blister, and preferably by more than 15% or 20% of the maximum lateral dimension of the blister.

6. A drug-delivery device according to any preceding claim, in which the dimensions of the outlet (12) and the inlets (38) are selected so that the rate of airflow inhaled by a user through the inhaler (2) is limited by airflow through the outlet.

7. A drug-delivery device according to any preceding claim, in which the depth of the blister (4) is greater than 1.2 times the distance which the air-outlet piercer (16) extends into the blister (4), and is preferably 1.5 or 2 or 3 times the distance which the air-outlet piercer extends into the blister.

8. A drug-delivery device according to any preceding claim, in which the air-outlet piercer (16) has a lateral dimension, and when the seal (10) is pierced, a cylindrical wall portion of the air-outlet piercer (16) extends into the blister (4) by a distance greater than half its lateral dimension so that no portion of the seal displaced by the air-outlet piercer impedes airflow through the outlet.

9. A drug-delivery device according to claim 8, in which the depth of the blister (4) is greater than 1.2 times the distance which the cylindrical wall portion of the air-outlet piercer (16) extends into the blister, and is preferably 1.5 or 2 or 3 times the distance which the cylindrical wall portion of the air-outlet piercer extends into the blister.

10. A drug-delivery device according to any preceding claim, in which one or more of the air-inlet piercers (30) comprises two walls (32, 34) meeting at an edge (36) for piercing the seal (10), such that the air-inlet piercer tapers outwardly from an upstream end to a downstream end at which an air-inlet (38) opening is defined between the walls so that, in use, air flowing within the air-inlet piercer, between the walls, enters the blister (4) through the air-inlet opening in a substantially tangential direction.

11. A drug-delivery device according to claim 10, in which the edge (36) between the walls (32, 34) of the air-inlet piercer (30) terminates at a point adjacent to the air-inlet (38) opening, so that penetration of the piercer through the seal (10) forms two substantially triangular portions of the seal lying alongside the walls of the piercer, which abut each other at their respective corners adjacent to the upstream end of the piercer.

12. A drug-delivery device according to claim 10 or 11, in which edges of the walls (32, 34) defining the air-inlet opening (38) are arranged substantially perpendicular to the seal (10) as the seal is pierced, so that no portion of the pierced seal impedes the flow of air through the air-inlet opening.

13. A drug-delivery device according to any preceding claim, in which all of the air inhaled by a user passes through the blister (4).

14. A drug-delivery device according to any preceding claim, in which, after use of the device, more than 80% or 90% of the carrier particles (62) are retained in the blister (4), and less than 20% or 10% of the API (64) is retained in the blister.

## Patentansprüche

1. Arzneimittelabgabevorrichtung, umfassend eine Inhalationsvorrichtung (2) und ein Blister (4), zur Desagglomeration, innerhalb des Blisters, eines pulverförmigen Medikaments (60), das einen pharmazeutischen Wirkstoff (API) (64) und mit dem API vermischte Trägerpartikel (62) umfasst, wobei:
das Blister das Medikament enthält und eine kreisförmige Blistertasche (6), die mit einem durchstechbaren Verschluss (10) verschlossen ist, umfasst; und
die Inhalationsvorrichtung Folgendes umfasst:
einen Luftauslass-Stecher (16) zum Stechen eines Luftauslasses (12) in den Verschluss, durch den Luft zu einem Inhalatormundstück gesaugt werden kann, wobei der Luftauslass axial in dem Blister positioniert ist; und
drei, vier oder fünf Lufteinlass-Stecher (30), die jeweils zum Stechen einer Einlassöffnung (38) in den Verschluss dienen, um Luft in das Blister zu leiten, wenn Luft durch den Auslass gesaugt wird, wobei jeder Einlass zwischen dem Luftauslass und einem Rand des Blisters positioniert ist und so geformt ist, dass im Gebrauch durch den Einlass gesaugte Luft in einer tangentialen Richtung in das Blister einströmt, um in dem Blister einen zyklonischen Luftstrom entstehen zu lassen, und so, dass ein stromaufwärtiges Ende jedes Lufteinlasses dem zyklonischen Luftstrom in dem Blister ein stromlinienförmiges Profil präsentiert;
wobei beim Durchstechen des Verschlusses ein oder mehrere der Lufteinlass-Stecher sich um weniger als die Entfernung, um die sich der Luftauslass-Stecher in das Blister hinein erstreckt, in das Blister hinein erstrecken;
wobei die Blistertasche (6) einen parabolischen Querschnitt hat, wobei die kreisförmige Blistertasche eine maximale seitliche Dimension hat und einer oder mehrere der Einlässe um mehr als 10 % der maximalen seitlichen Dimension des Blisters von einem Außenrand des Blisters und um mehr als 10 % der maximalen seitlichen Dimension des Blisters von einer Mittelachse des Blisters beabstandet sind; und wobei im Gebrauch der zyklonische Luftstrom innerhalb des Blisters das pulverförmige Medikament trägt und das pulverförmige Medikament innerhalb des Blisters radial nach außen beschleunigt, so dass der Durchfluss des API durch den Luftauslass relativ zum Durchfluss des Trägers durch den Auslass begünstigt wird.

2. Arzneimittelabgabevorrichtung nach Anspruch 1, wobei einer oder mehrere der Einlässe (38) um mehr als 15 % oder 20 % der maximalen seitlichen Dimension des Blisters von dem Außenrand des Blisters (4) beabstandet sind.

3. Arzneimittelabgabevorrichtung nach Anspruch 1 oder 2, wobei alle der Einlässe (38) um mehr als 10 % der maximalen seitlichen Dimension des Blisters und vorzugsweise um mehr als 15 % oder 20 % der maximalen seitlichen Dimension des Blisters von dem Außenrand des Blisters (4) beabstandet sind.

4. Arzneimittelabgabevorrichtung nach Anspruch 1, 2 oder 3, wobei einer oder mehrere der Einlässe (38) um mehr als 15 % oder 20 % der maximalen seitlichen Dimension des Blisters von der Mittelachse des Blisters (4) beabstandet sind.

5. Arzneimittelabgabevorrichtung nach einem der vorhergehenden Ansprüche, wobei alle der Einlässe (38) um mehr als 10 % der maximalen seitlichen Dimension des Blisters und vorzugsweise um mehr als 15 % oder 20 % der maximalen seitlichen Dimension des Blisters von der Mittelachse des Blisters (4) beabstandet sind.

6. Arzneimittelabgabevorrichtung nach einem der vorhergehenden Ansprüche, wobei die Dimensionen des Auslasses (12) und der Einlässe (38) so gewählt sind, dass die von einem Benutzer durch den Inhalator (2) eingeatmete Luftdurchflussrate durch den Luftstrom durch den Auslass begrenzt wird.

7. Arzneimittelabgabevorrichtung nach einem der vorhergehenden Ansprüche, wobei die Tiefe des Blisters (4) größer als das 1,2-fache der Entfernung ist, um die sich der Luftauslass-Stecher (16) in das Blister (4) hinein erstreckt, und vorzugsweise das 1,5-, 2- oder 3-fache der Entfernung ist, um die sich der Luftauslass-Stecher in das Blister hinein erstreckt.

8. Arzneimittelabgabevorrichtung nach einem der vorhergehenden Ansprüche, wobei der Luftauslass-Stecher (16) eine seitliche Dimension hat und, wenn der Verschluss (10) durchstochen ist, ein zylindrischer Wandteil des Luftauslass-Stechers (16) sich um eine größere Entfernung als die Hälfte seiner seitlichen Dimension in das Blister (4) hinein erstreckt, so dass kein Teil des von dem Luftauslass-Stecher verlagerten Verschlusses den Luftstrom durch den Auslass behindert.

9. Arzneimittelabgabevorrichtung nach Anspruch 8, wobei die Tiefe des Blisters (4) größer als das 1,2-fache der Entfernung ist, um die sich der zylindrische Wandteil des Luftauslass-Stechers (16) in das Blister hinein erstreckt, und vorzugsweise das 1,5-, 2- oder 3-fache der Entfernung ist, um die sich der zylindrische Wandteil des Luftauslass-Stechers in das Blister hinein erstreckt.

10. Arzneimittelabgabevorrichtung nach einem der vorhergehenden Ansprüche, wobei einer oder mehrere der Lufteinlass-Stecher (30) zwei Wände (32, 34) umfassen, die an einer Kante (36) zum Durchstechen des Verschlusses (10) aufeinandertreffen, so dass der Lufteinlass-Stecher von einem stromaufwärtigen Ende, an dem eine Lufteinlass- (38) -öffnung zwischen den Wänden definiert ist, nach außen breiter wird, so dass im Gebrauch in dem Lufteinlass-Stecher, zwischen den Wänden, fließende Luft durch die Lufteinlassöffnung in einer im Wesentlichen tangentialen Richtung in das Blister (4) eintritt.

11. Arzneimittelabgabevorrichtung nach Anspruch 10, wobei der Rand (36) zwischen den Wänden (32, 34) des Lufteinlass-Stechers (30) an einem Punkt endet, der an die Lufteinlass-(38) -öffnung angrenzt, so dass die Eindringung des Stechers durch den Verschluss (10) zwei im Wesentlichen dreieckige Teile des Verschlusses bildet, die an den Wänden des Stechers entlang liegen, die an ihren jeweiligen Ecken, die an das stromaufwärtige Ende des Stechers angrenzen, aneinanderstoßen.

12. Arzneimittelabgabevorrichtung nach Anspruch 10 oder 11, wobei Kanten der Wände (32, 34), die die Lufteinlassöffnung (38) definieren, beim Durchstechen des Verschlusses im Wesentlichen senkrecht zum Verschluss (10) angeordnet sind, so dass kein Teil des durchstochenen Verschlusses den Durchfluss von Luft durch die Lufteinlassöffnung hindert.

13. Arzneimittelabgabevorrichtung nach einem der vorhergehenden Ansprüche, wobei die gesamte von einem Benutzer inhalierte Luft durch das Blister (4) hindurchgeführt wird.

14. Arzneimittelabgabevorrichtung nach einem der vorhergehenden Ansprüche, wobei nach Gebrauch der Vorrichtung mehr als 80 % oder 90 % der Trägerteilchen (62) in dem Blister (4) zurückbehalten werden und weniger als 20 % oder 10 % des API (64) in dem Blister zurückbehalten werden.

## Revendications

1. Dispositif d'administration de médicament comprenant un appareil d'inhalation (2) et une cloque (4), pour désagglomérer, à l'intérieur de la cloque, un médicament en poudre (60) comprenant un ingrédient pharmaceutique actif (API) (64) et des particules porteuses (62) mélangées à l'API, dans lequel ;
la cloque contient le médicament et comprend une poche de cloque circulaire (6) scellée par un sceau perforable (10) ; et
l'appareil d'inhalation comprend :
un perforateur de sortie d'air (16) pour perforer une sortie d'air (12) à travers le sceau, au travers de laquelle l'air peut être attiré vers un embout buccal d'inhalateur, la sortie d'air étant positionnée axialement dans la cloque ; et
trois, quatre ou cinq perforateurs d'entrée d'air (30), chacun servant à perforer une ouverture d'entrée (38) à travers le sceau pour diriger l'air jusque dans la cloque lorsque l'air est aspiré à travers la sortie, chaque entrée étant positionnée entre la sortie d'air et un bord de la cloque, et façonnée de telle sorte, lors de l'utilisation, l'air attiré à travers l'entrée entre dans la cloque dans une direction tangentielle pour créer un écoulement d'air cyclonique à l'intérieur de la cloque, et de telle sorte qu'une extrémité amont de chaque entrée d'air présente un profil aérodynamique au flux d'air cyclonique à l'intérieur de la cloque ;
dans lequel lorsque le sceau est percé, un ou plusieurs des perforateurs d'entrée d'air s'étendent dans la cloque sur une distance inférieur à celle sur laquelle le perforateur de sortie d'air s'étend dans la cloque ;
dans lequel la poche de cloque (6) a une section transversale parabolique, dans lequel la poche de cloque circulaire a une dimension latérale maximale, et une ou plusieurs des entrées sont espacées d'un bord extérieur de la cloque par plus de 10 % de la dimension latérale maximale de la cloque, et d'un axe central de la cloque par plus de 10 % de la dimension latérale maximale de la cloque ; et dans lequel, lors de l'utilisation, l'écoulement d'air cyclonique à l'intérieur de la cloque convoie le médicament en poudre et accélère le médicament en poudre radialement vers l'extérieur à l'intérieur de la cloque, de telle sorte que l'écoulement de l'API à travers la sortie d'air soit favorisé par rapport à l'écoulement du porteur à travers la sortie.

2. Dispositif d'administration de médicament selon la revendication 1, dans lequel une ou plusieurs des entrées (38) sont espacées du bord extérieur de la cloque (4) par plus de 15 % ou 20 % de la dimension latérale maximale de la cloque.

3. Dispositif d'administration de médicament selon la revendication 1 ou 2, dans lequel toutes les entrées (38) sont espacées du bord extérieur de la cloque (4) par plus de 10 % de la dimension latérale maximale de la cloque, et de préférence par plus de 15 % ou 20 % de la dimension latérale maximale de la cloque.

4. Dispositif d'administration de médicament selon la revendication 1, 2 ou 3, dans lequel une ou plusieurs des entrées (38) sont espacées de l'axe central de la cloque (4) par plus de 15 % ou 20 % de la dimension latérale maximale de la cloque.

5. Dispositif d'administration de médicament selon toute revendication précédente, dans lequel toutes les entrées (38) sont espacées de l'axe central de la cloque (4) par plus de 10 % de la dimension latérale maximale de la cloque, et de préférence par plus de 15 % ou 20 % de la dimension latérale maximale de la cloque.

6. Dispositif d'administration de médicament selon toute revendication précédente, dans lequel les dimensions de la sortie (12) et des entrées (38) sont sélectionnées de telle sorte que le débit de l'écoulement d'air inhalé par un utilisateur à travers l'inhalateur (2) soit limité par l'écoulement d'air à travers la sortie.

7. Dispositif d'administration de médicament selon toute revendication précédente, dans lequel la profondeur de la cloque (4) est supérieure à 1,2 fois la distance sur laquelle le perforateur de sortie d'air (16) s'étend dans la cloque (4), et est de préférence 1,5, 2 ou 3 fois la distance sur laquelle le perforateur de sortie d'air s'étend dans la cloque.

8. Dispositif d'administration de médicament selon toute revendication précédente, dans lequel le perforateur de sortie d'air (16) a une dimension latérale, et une fois le sceau (10) percé, une partie de paroi cylindrique du perforeur de sortie d'air (16) s'étend dans la cloque (4) par une distance supérieure à la moitié de sa dimension latérale de telle sorte qu'aucune partie du sceau déplacée par le perforateur de sortie d'air n'empêche l'écoulement d'air à travers la sortie.

9. Dispositif d'administration de médicament selon la revendication 8, dans lequel la profondeur de la cloque (4) est supérieure à 1,2 fois la distance par laquelle la partie de paroi cylindrique du perforateur de sortie d'air (16) s'étend dans la cloque, et est de préférence 1,5, 2 ou 3 fois la distance sur laquelle la partie de paroi cylindrique du perforateur de sortie d'air s'étend dans la cloque.

10. Dispositif d'administration de médicament, selon toute revendication précédente, dans lequel un ou plusieurs des perforateurs d'entrée d'air (30) comprennent deux parois (32, 34) se rejoignant au niveau d'un bord (36) pour perforer le sceau (10), de telle sorte que le perforateur d'entrée d'air s'effile vers l'extérieur depuis une extrémité amont vers une extrémité aval à laquelle une ouverture d'entrée d'air (38) est définie entre les parois de telle sorte que, lors de l'utilisation, l'air s'écoulant à l'intérieur du perforateur d'entrée d'air, entre les parois, entre dans la cloque (4) à travers l'ouverture d'entrée d'air dans une direction sensiblement tangentielle.

11. Dispositif d'administration de médicament selon la revendication 10, dans lequel le bord (36) entre les parois (32, 34) du perforateur d'entrée d'air (30) se termine au niveau d'un point adjacent à l'ouverture de l'entrée d'air (38), de telle sorte que la pénétration du perforateur à travers le sceau (10) forme deux parties sensiblement triangulaires du sceau reposant le long des parois du perforateur, lesquelles se jouxtent l'une l'autre au niveau de leurs coins respectifs adjacents à l'extrémité amont du perforateur.

12. Dispositif d'administration de médicament selon la revendication 10 ou 11, dans lequel des bords des parois (32, 34) définissant l'ouverture d'entrée d'air (38) sont disposés de manière sensiblement perpendiculairement au sceau (10) lorsque le sceau est percé, de telle sorte qu'aucune partie du sceau percé n'entrave l'écoulement d'air à travers l'ouverture d'entrée d'air.

13. Dispositif d'administration de médicament selon toute revendication précédente, dans lequel tout l'air inhalé par un utilisateur passe à travers la cloque (4).

14. Dispositif d'administration de médicament selon toute revendication précédente, dans lequel, après l'utilisation du dispositif, plus de 80 % ou 90 % des particules porteuses (62) est retenu dans la cloque (4), et moins de 20 % ou 10 % de l'API (64) est retenu dans la cloque.
